Europäisches Patentamt

European Patent Office

Office européen des brevets

(19)

(11) Publication number: **0 165 470**
A2

(12) **EUROPEAN PATENT APPLICATION**

(21) Application number: **85106014.5**

(22) Date of filing: **15.05.85**

(51) Int. Cl.⁴: **C 07 C 15/02**
C 07 C 15/04, C 07 C 15/06
C 07 C 5/02, B 01 J 29/32

(30) Priority: **21.05.84 JP 100548/84**

(43) Date of publication of application:
**27.12.85 Bulletin 85/52**

(84) Designated Contracting States:
**DE FR GB IT NL**

(71) Applicant: **IDEMITSU PETROCHEMICAL COMPANY LIMITED**
No. 1-1, 3-chome, Marunouchi Chiyoda-ku
Tokyo(JP)

(72) Inventor: **Yamada, Tadashi**
No. 8-2, Higashiyama-cho
Tokuyama-shi Yamaguchi-ken(JP)

(72) Inventor: **Aoki, Akinobu**
No. 6701, Oaza-Tokuyama
Tokuyama-shi Yamaguchi-ken(JP)

(74) Representative: **Türk; Dietmar, Dr. rer. nat. et al,**
Redies, Redies, Türk & Gille Patentanwälte
Brucknerstrasse 20
D-4000 Düsseldorf 13(DE)

(54) **A method for the preparation of benzene and methyl-substituted benzene derivatives.**

(57) The invention provides an improvement in the yield of benzene and methyl-substituted benzene derivatives such as toluene, xylene and trimethylbenzenes by the catalytic hydrogenation treatment of heavy aromatic hydrocarbon compounds having 9 or more carbon atoms in a molecule. The improvement comprises the use of a specific solid catalyst which is a crystalline aluminosilicate supporting a metallic element selected from the metallic elements belong-ing to the VIIIth Group of the Periodic Table including nickel, platinum, palladium, rhodium and iridium introduced into the carrier by the ion exchange treatment. The hydrogenation treatment of the heavy aromatic hydrocarbon compound is effective to selectively dealkylating the alkyl groups other than methyl.

FIG. 1.

EP 0 165 470 A2

# A METHOD FOR THE PREPARATION OF BENZENE AND METHYL-SUBSTITUTED BENZENE DERIVATIVES

## BACKGROUND OF THE INVENTION

The present invention relates to a method for the preparation of benzene and methyl-substituted benzene derivatives or, more particularly, to an efficient method for the preparation of benzene and methyl-substituted benzene derivatives having high usefulness in industry starting from heavy aromatic hydrocarbons.

. As is known, the separation process by distillation or crystallization is conventionally practiced in the prior art in the preparation of industrially useful aromatic compounds having about 9 or 10 carbon atoms in a molecule from a heavy oil and the like as the starting material. Due to the proximity of the boiling points of many of the useful aromatic compounds to each other, however, the separation process by distillation must be performed using distillation columns of a large number of plates with very large energy consumption so that the process is economically disadvantageous with very high production costs.

Therefore, a method is proposed for the preparation of useful light aromatics by the selective dealkylation of the heavy aromatics. The dealkylation reaction hitherto undertaken is, however, not sufficient as a complete solution of the above mentioned problem due to the extremely low conversion.

## SUMMARY OF THE INVENTION

An object of the present invention is therefore to provide an efficient method for the preparation of useful aromatic compounds from heavy aromatics with low production costs.

Another object of the present invention is to provide an efficient method for the preparation of benzene and methyl-substituted benzene derivatives by the hydrogenation of heavier aromatic hydrocarbon compounds having 9 or more carbon atoms in a molecule in the presence of a specific catalyst.

Thus, the method of the present invention for the preparation of benzene and methyl-substituted benzene derivatives comprises hydrogenating a heavy aromatic hydrocarbon compound having at least 9 carbon atoms in a molecule in the presence of a crystalline aluminosilicate catalyst supporting at least one metallic element selected from the metallic elements belonging to Group VIII of the Periodic Table. The metallic element supported on the crystalline aluminosilicate carrier is preferably selected from the class consisting of nickel, platinum, palladium, rhodium and iridium.

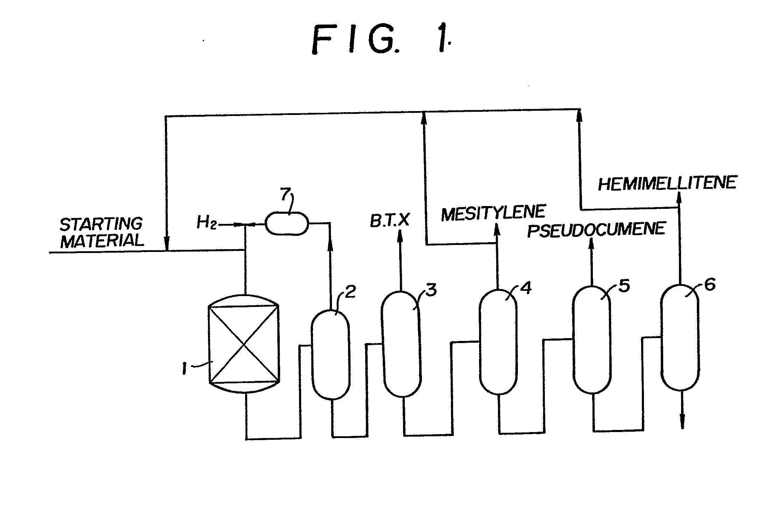

## BRIEF DESCRIPTION OF THE DRAWING

The figure is a schematic illustration of the apparatus used in the Examples for the hydrogenation of the heavy aromatics and separation of the products.

- 2 -

DETAILED DESCRIPTION OF THE PREFERRED EMBODIMENTS 0165470

The starting material used in the inventive method is a heavy aromatic hydrocarbon compound having at least 9 carbon atoms in a molecule. Such a material is exemplified, for example, by the heavy oils obtained in the distillation or reforming of crude oils and the heavy oils obtained in the process for the manufacture of benzene, toluene, xylene and the like from naphtha. The constituent aromatic compounds in these heavy oils include ethyltoluenes, trimethylbenzenes, diethylbenzenes, ethylxylenes, propylbenzenes, tetramethylbenzenes and the like. The trimethylbenzenes are particularly useful as the starting material in the inventive method among the above named aromatic hydrocarbon compounds while they are used hitherto mainly as a fuel because difficulties are encountered in the separation of them by distillation due to the proximity of their boiling points to those of the other constituent compounds.

The hydrogenation reaction in the inventive method is performed in the presence of a solid catalyst which is a crystalline aluminosilicate catalyst supporting at least one metallic element selected from the metallic elements belonging to Group VIII of the Periodic Table including nickel, platinum, palladium, rhodium, iridium, ruthenium, iron, cobalt and osmium, of which the former five elements are preferable. These metallic elements can be used either singly or as a combination of two kinds or more according to need.

The type of the crystalline aluminosilicate as the carrier of the catalyst used in the inventive method is not particularly limitative but is typically exemplified by ZSM-5, ZSM-11, ZSM-12, ZSM-23, ZSM-35, ZSM-38, ZSM-48 and the like. Various methods are known for the preparation of these crystalline aluminosilicates, in which a hydrothermal reaction is performed in an aqueous medium with addition of a variety of silica source materials, alumina source materials and crystallizing agents. For example, following is a description of the method for the preparation of an ammonium form crystalline aluminosilicate.

Thus, three aqueous solutions A, B and C are prepared by dissolving aluminum sulfate, sulfuric acid and tetrapropylammonium bromide in water for the solution A, water glass composed of silicon oxide and sodium oxide in water for the solution B and sodium chloride in water for the solution C. The solutions A and B are added dropwise to the solution C to form a reaction mixture which, after adjustment of the pH to a suitable value according to need, is heated in an autoclave under hydrothermal conditions to precipitate crystalline aluminosilicate. The precipitates separated from the reaction mixture after completion of the reaction are washed with water, dried and calcined to give a crystalline aluminosilicate of sodium form which is then converted into the ammonium form by the treatment with an aqueous solution of ammonium nitrate. The crystalline aluminosilicate obtained in this manner is usually in a powdery form but it is optional to shape the powder into a granular form by use of a binder such as an alumina sol, if desired.

- 4 -

The solid catalyst used in the inventive method is prepared by supporting the thus obtained crystalline aluminosilicate as the carrier with one or more of the metallic elements selected from those belonging to Group VIII of the Periodic Table as is mentioned above. The method for impregnating the carrier with the metallic element is not particularly limitative including various known methods such as the impregnating method, ion exchange method and the like. It is also optional that the impregnation of the carrier with the metallic element is carried out on the crystalline aluminosilicate in the powdery form as such followed by shaping into a desired granular form.

The solid catalysts prepared in the above described manner, though diversified in the catalytic performance, can be used in the method of the present invention without particular limitations. It is, however, preferable that the molar ratio of silica to alumina in the crystalline aluminosilicate is in the range from 1 to about 150 or, more preferably, from 1 to about 100. The amount of the metallic element supported on the crystalline aluminosilicate carrier is also not particularly limitative, usually, in the range from 0.001 to 5.0% by weight although the optimum content of the metallic element depends on the kind of the element. For example, the content of nickel in the catalyst should preferably be in the range from 0.1 to 5.0% by weight or, more preferably, from 0.5 to 3.0% by weight while the content of platinum, palladium, rhodium or iridium should preferably be in the range from 0.001 to 1.0% by weight or, more preferably, in the range from 0.01 to 1.0% by weight.

The method of the present invention essentially comprises the hydrogenation treatment of the above mentioned heavy aromatic hydrocarbon compound in the presence of the solid catalyst described above. This hydrogenation treatment has an effect of selective dealkylation of the alkyl groups other than methyl, such as ethyl group, propyl group and the like, bonded to the aromatic nucleus of the heavy aromatic hydrocarbon compound as the starting material.

Although the conditions of the hydrogenation reaction in the inventive method are not particularly limitative, a rough guideline therefor includes a pressure of the reaction system in the range from atmospheric pressure to 20 kg/cm$^2$G or, preferably, from atmospheric pressure to 12 kg/cm$^2$G, a weight hourly space velocity (WHSV) in the range from 0.1 to 20 hour$^{-1}$ or, preferably, from 0.2 to 4 hour$^{-1}$ and a molar ratio of hydrogen gas to the heavy aromatic hydrocarbon compound as the starting material in the range from 0.5 to 20 or, preferably, from about 3 to about 10. In connection with the temperature for the hydrogenation reaction, on the other hand, no single guideline can be given depending on the kind of the metallic element supported on the crystalline aluminosilicate carrier. For example, the temperature should be in the range from 250 to 550°C or, preferably, from 300 to 400°C when the active metallic element in the solid catalyst is nickel while the temperature should be in the range from 420 to 550°C or, preferably, from 450 to 500°C when the catalyst contains platinum, palladium, rhodium or iridium as the active

- 6 -

metallic ingredient.

The hydrogenation reaction of the inventive method can be performed in a variety of apparatuses used in similar reactions. For example, a relatively simple apparatus schematically illustrated in the accompanying drawing can be used satisfactorily and the individual components in the reaction products can be separated without using a distillation column with a large number of plates.

As a result of the selective dealkylation of the alkyl groups other than methyl, e.g. ethyl group, bonded to the benzene nucleus, the product of the hydrogenation treatment of the heavy aromatic hydrocarbon compound in the presence of the above described specific catalyst according to the inventive method is mainly composed of benzene, toluene and xylene. Further, when a starting heavy aromatic hydrocarbon mixture containing ethyltoluenes, trimethylbenzenes, diethylbenzenes, ethylxylenes and the like hardly separable by distillation due to the proximity of their boiling points is subjected to the hydrogenation treatment according to the inventive method, the ethyltoluenes, diethylbenzenes and ethylxylenes are de-ethylated to give benzene, benzene and xylenes, respectively, to leave the trimethylbenzenes unreacted so that removal of these de-ethylated aromatic compounds from the reaction mixture after the reaction gives a product rich in the content of the industrially useful methyl-substituted benzene derivatives such as trimethylbenzenes. In addition, high-purity products of the methyl-substituted benzene derivatives such as trimethylbenzenes

- 7 -

can readily be obtained by a simple distillation for purification of the reaction mixture after the hydrogenation treatment by virtue of the extreme decrease of the contents of the
species having a boiling point close to that of the trimethylbenzenes and other desired compounds.

As is described above, the method of the present invention is useful when it is desired to obtain a reaction product
containing large amounts of benzene and methyl-substituted
aromatic hydrocarbon compounds such as toluene, xylenes,
trimethylbenzenes and the like having a high industrial value
starting from heavy aromatics hardly separable by the conventional method of separation such as distillation and the
like. Furthermore, each of the above named methyl-substituted
aromatic hydrocarbon compounds has a boiling point widely
different from those of the other compounds in the reaction
mixture so that these compounds can readily be separated into
the individual component compounds of high purity even by a
purification method of simple distillation.

As is understood from the above description, the method
of the present invention is very useful in the petrochemical
industry since the invention provides a highly efficient
method for the preparation of benzene and methyl-substituted
benzene derivatives starting from heavy aromatics having a
relatively low industrial value.

In the following, the method of the present invention
is illustrated in more detail by way of examples preceded
by a description of the preparation procedure of the catalyst
used in each of the Examples.

Catalyst Preparation 1.

An aqueous reaction mixture was prepared by concurrently adding dropwise a first aqueous solution prepared by dissolving 6.2 g of aluminum sulfate octadecahydrate $Al_2(SO_4)_3 \cdot 18H_2O$, 17.68 g of concentrated sulfuric acid and 26.32 g of tetrapropylammonium bromide in 250 ml of water and a second aqueous solution prepared by dissolving 211.1 g of a water glass having a composition of 28.95% by weight of $SiO_2$, 9.40% by weight of $Na_2O$ and 61.65% by weight of $H_2O$ in 250 ml of water into a third aqueous solution prepared by dissolving 79.0 g of sodium chloride in 222 ml of water at room temperature over a period of 60 minutes. After adjustment of the pH to 9.5 using sulfuric acid, the thus prepared aqueous solution was introduced into an autoclave and heated at 170°C for 20 hours under autogenous pressure.

After cooling, the reaction mixture was taken out of the autoclave and the precipitates collected by filtration were washed with water and then subjected to three times of ion exchange treatment at 90°C for 8 hours with 300 ml of a 1N aqueous solution of ammonium nitrate to give a crystalline aluminosilicate of ammonium form.

Further, the solid material collected by filtration and washed with water was dried at 120°C for 24 hours and then calcined at 550°C for 6 hours to give a crystalline aluminosilicate of H form. This material was admixed with an alumina sol as a binder in such an amount that the content of the binder in the mixture was 20% by weight and the mixture was shaped into pellets, dried at 120°C for 3 hours and calcined in air at 550°C for 6 hours to give pellets of the crystalline aluminosilicate of H form.

The thus obtained crystalline aluminosilicate in the form of pellets was further calcined in air at 450°C for 16 hours in an electric muffle furnace and a 20 g portion of the calcined pellets was subjected to an ion exchange treatment by dipping in 200 ml of a 5% by weight aqueous solution of ammonium chloride at 80°C for 24 hours. Then, the solid material was thoroughly washed with water, dried at 100°C for 8 hours and calcined in air at 450°C for 16 hours in an electric muffle furnace.

A 10 g portion of the thus obtained calcined material was subjected to an ion exchange treatment with nickel ions by dipping in an aqueous solution prepared by dissolving 0.51 g of hexahydrated nickel nitrate in 30 ml of water at 50°C for 24 hours followed by evaporation of water with agitation. Thereafter, the solid material was dried at 200°C for 4 hours and calcined in air at 450°C for 16 hours in an electric muffle furnace. The content of nickel in the thus obtained powdery nickel-supporting crystalline aluminosilicate was 1.1% by weight.

The above obtained powdery crystalline aluminosilicate was then admixed and thoroughly blended with an equal amount of alumina gel for chromatographic use and the powdery blend was shaped into granules having a 10 to 20 mesh particle size distribution followed by calcination at 450°C for 16 hours to give a final product of the catalyst.

Examples 1 to 3.

A reactor column 1 for the fixed-bed continuous reaction in the bench scale apparatus illustrated in the figure of the accompanying drawing was filled with the catalyst prepared in the above described Catalyst Preparation 1, in which the hydrogenation treatment was undertaken with a starting material composed of 50.0% by weight of ethyltoluene, 40.0% by weight of trimethylbenzene, 5.0% by weight of diethylbenzene and 5.0% by weight of ethylxylene under the reaction conditions summarized in Table 1 given below. The reaction mixture coming out of the reactor column in each of the Examples was separated into the individual component hydrocarbon compounds by passing through the stripper 2 and the distillation columns 3, 4, 5 and 6. The results are shown also in Table 1.

In Example 3, 10.000 kg of the reaction mixture coming out of the reactor column were introduced at room temperature under atmospheric pressure into the stripper 2 and 1.810 kg of a gaseous mixture of hydrogen.and $C_1$ to $C_5$ paraffins rich in the content of hydrogen were recovered at the column top of the stripper and recycled to the reactor column 1. On the other hand, 8.190 kg of the liquid recovered at the column bottom of the stripper 2 were introduced into the first distillation column 3, from the column top of which 5.470 kg of a liquid mixture composed of 0.930 kg of benzene, 3.000 kg of toluene and 1.540 kg of xylene were taken out. On the other hand, 2.720 kg of the liquid recovered from the bottom of the first distillation column 3 were introduced

- 11 -

into the second distillation column 4, from the column top of which 0.020 kg of ethyltoluene and 0.64 kg of 1,3,5-trimethylbenzene, i.e. mesitylene, were taken out and returned to the reactor column 1. On the other hand, 2.060 kg of the liquid recovered from the bottom of the second distillation column 4 were introduced into the third distillation column 5, from the column top of which a liquid mixture composed of 0.010 kg of 1,3,5-trimethylbenzene, 1.700 kg of 1,2,4-trimethylbenzene, i.e. pseudocumene, and 0.010 kg of 1,2,3-trimethylbenzene, i.e. hemimellitene, was obtained. On the other hand, 0.340 kg of the liquid recovered from the bottom of the third distillation column 5 was introduced into the fourth distillation column 6, from the column top of which 0.250 kg of 1,2,3-trimethylbenzene and 0.010 kg of ethylxylene were taken out and returned to the reactor column 1. On the other hand, 0.010 kg of 1,2,3-trimethylbenzene and 0.070 kg of ethylxylene were obtained from the bottom of the fourth distillation column 6.

Following is a tabulation of the operation conditions of each of the distillation columns.

| Distillation column | 1st | 2nd | 3rd | 4th |
|---|---|---|---|---|
| Number of plates | 30 | 150 | 90 | 50 |
| Temperature at column bottom, °C | 197 | 201 | 211 | 220 |
| Temperature at column top, °C | 116 | 163 | 168 | 170 |
| Pressure at column bottom, kg/cm$^2$ abs | 2 | 2 | 2 | 2 |
| Pressure at column top, kg/cm$^2$ abs | 1 | 1 | 1 | 1 |
| Reflux ratio | 1.0 | 32.0 | 8.0 | 6.0 |

Catalyst Preparation 2.

An aqueous reaction mixture was prepared by concurrently adding dropwise a first aqueous solution prepared by dissolving 6.2 g of hydrated aluminum sulfate $Al_2(SO_4)_3 \cdot 18H_2O$, 17.68 g of concentrated sulfuric acid and 26.32 g of tetrapropylammonium bromide in 250 ml of water and a second aqueous solution prepared by dissolving 211.1 g of a water glass having a composition of 28.95% by weight of $SiO_2$, 9.40% by weight of $Na_2O$ and 61.65% by weight of $H_2O$ in 250 ml of water into a third aqueous solution prepared by dissolving 79.0 g of sodium chloride in 222 ml of water at room temperature over a period of 60 minutes. After adjustment of the pH to 9.5 using sulfuric acid, the thus prepared aqueous solution was introduced into an autoclave and heated at 170°C for 20 hours under autogenous pressure.

After cooling, the reaction mixture was taken out of the autoclave and the precipitates collected by filtration were washed with water and then subjected to three times of ion exchange treatment at 90°C for 8 hours with 300 ml of a 1N aqueous solution of ammonium nitrate to give a crystalline aluminosilicate of ammonium form.

Further, the solid material collected by filtration and washed with water was dried at 120°C for 24 hours and then calcined at 550°C for 6 hours to give a crystalline aluminosilicate of H form. This material was admixed with an alumina sol as a binder in such an amount that the content of the binder in the mixture was 20% by weight and the mixture was shaped into pellets, dried at 120°C for 3 hours and calcined

- 13 -

Table 1

| Example No. | | 1 | 2 | 3 |
|---|---|---|---|---|
| Reaction condi- tions | Reaction temperature, °C | 365 | 390 | 415 |
| | Reaction pressure, $kg/cm^2G$ | 5 | 5 | 5 |
| | Molar ratio of hydrogen/hydrocarbons | 4 | 4 | 4 |
| | WHSV, $hour^{-1}$ | 1 | 1 | 1 |
| Composi- tion of product, % by weight | $C_1$ to $C_5$ paraffins | 14.6 | 16.6 | 18.1 |
| | Benzene | 7.0 | 8.5 | 9.3 |
| | Toluene | 27.7 | 30.6 | 30.0 |
| | Ethylbenzene | 3.1 | 0.4 | 0.0 |
| | Xylene | 4.9 | 9.6 | 15.4 |
| | Ethyltoluene | 3.6 | 0.7 | 0.2 |
| | Trimethylbenzene | 37.2 | 32.7 | 26.2 |
| | Diethylbenzene | 0.1 | 0.0 | 0.0 |
| | Ethylxylene | 1.8 | 0.9 | 0.8 |
| Yield of aromatics, %[1] | | 99.5 | 99.5 | 99.0 |
| ET (ethyltoluene) decomposition, %[2] | | 92.8 | 98.6 | 99.6 |
| TMB (trimethylbenzene) decomposition, %[3] | | 7.0 | 18.3 | 34.5 |

[1]: (moles of aromatics in product/moles of aromatics in feed) x 100

[2]: $\dfrac{\text{(moles of ET in feed - moles of ET in product)}}{\text{moles of ET in feed}}$ x 100

[3]: $\dfrac{\text{(moles of TMB in feed - moles of TMB in product)}}{\text{moles of TMB in feed}}$ x 100

in air at 550°C for 6 hours to give pellets of the crystalline aluminosilicate of H form.

The thus obtained crystalline aluminosilicate in the form of pellets was further calcined in air at 450°C for 16 hours in an electric muffle furnace and a 20 g portion of the calcined pellets was subjected to an ion exchange treatment by dipping in 200 ml of a 5% by weight aqueous solution of ammonium chloride at 80°C for 24 hours. Then, the solid material was thoroughly washed with water, dried at 100°C for 8 hours and calcined in air at 450°C for 16 hours in an electric muffle furnace.

A 10 g portion of the thus obtained calcined material was subjected to an ion exchange treatment with platinum ions by dipping in an aqueous solution prepared by dissolving 0.052 g of dichloroammonium platinum in 30 ml of water at 50°C for 8 hours. The solid material collected by filtration was thoroughly washed with water and then dried in an electric drying oven first at 100°C for 8 hours and then at 200°C for 16 hours to give a platinum-containing crystalline aluminosilicate, of which the content of platinum was 0.2% by weight.

The above obtained platinum-containing crystalline aluminosilicate was then admixed and thoroughly blended with an equal amount of alumina gel for chromatographic use and the blend was shaped into granules having a 10 to 20 mesh particle size distribution followed by calcination at 450°C for 16 hours to give a final product of the catalyst.

Examples 4 and 5.

The same reactor column as used in the preceding Examples was filled with the platinum-supporting catalyst prepared in the above described Catalyst Preparation 2 and the hydrogenation reaction of the heavy aromatic hydrocarbon mixture was performed in this reactor column under the conditions shown in Table 2 shown below. The composition of the starting heavy aromatic hydrocarbon mixture was the same as that of the mixture used in the preceding examples. The reaction mixture coming out of the reactor column was separated into the individual component hydrocarbon compounds by passing through the stripper 2 and the distillation columns 3, 4, 5 and 6. The results are shown in Table 2.

In Example 5, 10.000 kg of the reaction mixture coming out of the reactor column were introduced at room temperature under atmospheric pressure into the stripper 2 and 1.660 kg of a gaseous mixture of hydrogen and $C_1$ to $C_5$ paraffins rich in the content of hydrogen were recovered at the column top of the stripper and recycled to the reactor column 1. On the other hand, 8.340 kg of the liquid recovered at the column bottom of the stripper 2 were introduced into the first distillation column 3, from the column top of which 4.970 kg of a liquid mixture composed of 0.810 kg of benzene, 3.100 kg of toluene and 1.060 kg of xylene were taken out. On the other hand, 3.370 kg of the liquid recovered from the bottom of the first distillation column 3 were introduced into the second distillation column 4, from the column top of which 0.100 kg of ethyltoluene and 0.785 kg of 1,3,5-trimethylbenzene,

- 16 -

i.e. mesitylene were taken out and returned to the reactor column 1. On the other hand, 2.485 kg of the liquid recovered from the bottom of the second distillation column 4 were introduced into the third distillation column 5, from the column top of which a liquid mixture composed of 0.010 kg of 1,3,5-trimethylbenzene, 2.067 kg of 1,2,4-trimethylbenzene, i.e. pseudocumene, and 0.010 kg of 1,2,3-trimethylbenzene, i.e. hemimellitene, was obtained. On the other hand, 0.398 kg of the liquid recovered from the bottom of the third distillation column was introduced into the fourth distillation column 6, from the column top of which 0.298 kg of 1,2,3-trimethylbenzene and 0.010 kg of ethylxylene were taken out and returned to the reactor column 1. On the other hand, 0.010 kg of 1,2,3-trimethylbenzene and 0.080 kg of ethylxylene were obtained from the bottom of the fourth distillation column 6.

Following is a tabulation of the operation conditions of each of the distillation columns.

| Distillation column | 1st | 2nd | 3rd | 4th |
|---|---|---|---|---|
| Number of plates | 30 | 150 | 90 | 50 |
| Temperature at column bottom, °C | 197 | 201 | 211 | 220 |
| Temperature at column top, °C | 116 | 163 | 168 | 170 |
| Pressure at column bottom, kg/cm$^2$ abs | 2 | 2 | 2 | 2 |
| Pressure at column top, kg/cm$^2$ abs | 1 | 1 | 1 | 1 |
| Reflux ratio | 1.0 | 32.0 | 8.0 | 6.0 |

Comparative Example.

The experimental procedure for the hydrogenation treatment was the same as in the above described Examples 4 and 5 except that the solid catalyst filling the reactor column was the crystalline aluminosilicate supporting no metallic element introduced by ion exchange. The results of the experiment are shown in Table 2.

Table 2

| | Example No. | 4 | 5 | Comparative Example |
|---|---|---|---|---|
| Reaction condition | Reaction temperature, °C | 450 | 490 | 400 |
| | Reaction pressure, $kg/cm^2 G$ | 12 | 12 | 12 |
| | Molar ratio of hydrogen/hydrocarbon | 4 | 4 | 4 |
| | WHSV, $hour^{-1}$ | 2 | 2 | 2 |
| Composition of product, % by weight | $C_1$ to $C_5$ paraffins | 15.8 | 16.6 | 7.6 |
| | Benzene | 7.7 | 8.1 | 3.9 |
| | Toluene | 29.9 | 31.0 | 21.5 |
| | Ethylbenzene | 0.5 | 0.2 | 2.3 |
| | Xylene | 8.1 | 10.4 | 3.5 |
| | Ethyltoluene | 2.6 | 1.0 | 20.0 |
| | Trimethylbenzene | 34.4 | 31.8 | 36.7 |
| | Diethylbenzene | 0.0 | 0.0 | 1.5 |
| | Ethylxylene | 1.0 | 0.9 | 3.0 |
| Yield of aromatics, %[1] | | 99.5 | 99.5 | 99.5 |
| ET (ethyltoluene) decomposition, %[2] | | 94.8 | 98.0 | 60.0 |
| TMB (trimethylbenzene) decomposition, %[3] | | 14.0 | 20.5 | 8.3 |

[1], [2] and [3]: See footnotes to Table 1.

WHAT IS CLAIMED IS:

1. A method for the preparation of benzene and methyl-substituted benzene derivatives which comprises hydrogenating a heavy aromatic hydrocarbon compound having at least 9 carbon atoms in a molecule in the presence of a crystalline aluminosilicate catalyst supporting at least one metallic element selected from the metallic elements belonging to Group VIII of the Periodic Table.

2. The method as claimed in claim 1, wherein the metallic element is selected from the class consisting of nickel, platinum, palladium, rhodium and iridium.

3. The method as claimed in claim 2, wherein the amount of nickel supported on the crystalline aluminosilicate is in the range from 0.1 to 5.0% by weight based on the amount of the crystalline aluminosilicate.

4. The method as claimed in claim 1, wherein the metallic element is selected from the class consisting of platinum, palladium, rhodium and iridium and the amount of the metallic element is in the range from 0.001 to 1.0% by weight based on the amount of the crystalline aluminosilicate.

5. The method as claimed in claim 1, wherein the molar ratio of silica to alumina in the crystalline aluminosilicate is in the range from 1 to 150.

# FIG. 1

STARTING MATERIAL · H₂ · 7 · B.T.X · MESITYLENE · PSEUDOCUMENE · HEMIMELLITENE · 1 · 2 · 3 · 4 · 5 · 6